Europäisches Patentamt

(19) **European Patent Office**

**Office européen des brevets**

(11) Publication number: **0 211 991**
**B1**

(12) # EUROPEAN PATENT SPECIFICATION

(45) Date of publication of the patent specification:
**25.10.89**

(21) Application number: **85110591.6**

(22) Date of filing: **23.08.85**

(51) Int. Cl.⁴: **A61K 9/32**

(54) Substained release tablets and method for preparation thereof.

(43) Date of publication of application:
**04.03.87 Bulletin 87/10**

(45) Publication of the grant of the patent:
**25.10.89 Bulletin 89/43**

(84) Designated Contracting States:
**AT BE CH DE FR GB IT LI LU NL SE**

(56) References cited:
**EP-A- 0 096 074**
**EP-A- 0 113 776**
**FR-A- 2 364 660**
**GB-A- 1 186 990**
**US-A- 3 538 214**
**US-A- 3 595 939**
**US-A- 3 954 959**
**US-A- 4 060 598**
**US-A- 4 557 925**

**CHEMICAL ABSTRACTS, vol. 83, no. 22, 1st**
**December 1975, page 72, no. 180527x, Columbus, Ohio,**
**US; L. BONNET: "Process for making microporous**
**membranes", & RES. DISCL. 1975, 135, 74-5**

The file contains technical information submitted after
the application was filed and not included in this
specification

(73) Proprietor: **AB LEO, Box 941, S-251 09 Helsingborg(SE)**

(72) Inventor: **Erlandsson, Stig Albin Bertil, Fagusgatan 11,**
**S-212 32 Malmö(SE)**
Inventor: **Lindahl, Ake Rikard, Ringduvevägen 50,**
**S-274 00 Skurup(SE)**

(74) Representative: **Thylén, Eva, c/o AB Leo Patent**
**Department Box 941, S-251 09 Helsingborg(SE)**

## Description

The sustained release of a medicine or drug is important for several reasons. For example it serves to provide the body with medication over a long time and thereby eliminates the need for swallowing an ordinary tablet at frequent intervals. Another advantage could be that a sudden release of a large amount of medicine which often occurs when conventional tablets are used can be avoided. Thereby the onset of toxic symptoms can be eliminated.

One type of commercially used slow release tablets comprises a firmly coherent skeleton structure composed of non-toxic synthetic resin substantially insoluble in the fluid of the human or animal body with which the tablet comes into contact. The structure includes pore-like inter-connected canals or ducts open to the exterior of the structure and a material which comprises or contains a medical substance, which is soluble in the fluid contained in such canals or ducts. Slow release tablets of this type are disclosed in the GB patent 808,014.

Another type of commercially used slow release preparation known as "osmotic device" has been developed by Alza Co. This device comprises a wall surrounding a reservoir containing an active agent and having a passageway for releasing the agent from the device. The wall is comprised of a material permeable to external fluid.

A third type of slow release tablet is disclosed in e.g. the GB patent 1,186,990 and US patent 3,538,214. These patents disclose pharmaceutical preparations consisting of a tablet core comprising a medicament, which is soluble in the gastro-intestinal fluids, and a coating on said core. The coating consists of a polymer substance which remains substantially intact and insoluble in the gastro-intestinal fluids. Fine particles of a readily water-soluble substance are randomly distributed in the coating. Furthermore, it is disclosed in the patent that the preparation can be provided with an additional coating which i.a. may contain another pharmacologically active substance.

According to the GB patent 1,186,990 the polymer substance is a polyamide and according to the US patent 3,538,314 the polymer substance is cellulose acetate, ethyl cellulose or low water soluble polyvinyl alcohol.

However, to the best of our knowledge, none of the technical solutions disclosed in these two patents has led to a practically useful product. As regards the US patent 3,538,214 the reason seems to be clear, as the tables of the dissolution rate disclose that the dissolution rate is neither pH-independent nor essentially constant over any longer period of time, two requirements that must be fulfilled in modern slow release preparations. The polymer substance used in the GB patent 1,186,990 has rather poor film-forming properties, and it is difficult to get a proper adhesion to the tablet core surface.

Another slow release composition is disclosed in the US patent 4,244,941. This composition comprises a drug including a core of a soluble substance and a rigid porous coating completely surrounding the core. The coating is substantially free of substances soluble or swellable in the gastrointestinal liquids. Furthermore, the coating is selected from substances insoluble in the medium, in which they are intended to be used. The substances are compressed in powder form to form an inert non-disintegrating, noneroding porous coating on the core. The coating could e.g. consist of a copolymer of vinyl chloride and vinyl acetate.

However, this compression coating principle produces tablets, which consist of up to about 50% of coating material. This is contrary to what is sought after in the medical and pharmaceutical fields today.

### Objects of the invention

An object of the present invention is to provide a slow-release preparation, in which the dissolution rate of the active substance is substantially constant over a long period of time. This means that the rate determining characteristics of the coating is substantially independent of any mechanical influence, enzymes, surface tension, pH and salt concentration.

Another object of the invention is to provide an inexpensive slow release preparation.

A third object is to provide a slow release preparation generally useful for different kinds of orally active drugs.

A fourth object is to provide a slow release preparation, which is small in size due to a favourable ratio between the amount of active substance and inactive ingredients.

A further object of the invention is to provide a slow release preparation, which can be manufactured in a simple process without problems with roughness of the tablet surface ("orange peel") or other problems related to poor adhesion of the coating to the tablet core surface.

### Summary of the invention

A slow-release tablet according to the present invention comprises a drug containing tablet and a coating surrounding the same. The coating is insoluble in water and in the gastrointestinal fluids and consists essentially of a terpolymer of polyvinyl chloride, polyvinyl acetate and polyvinyl alcohol and a pore-creating substance being randomly distributed in the terpolymer. The pore-creating substance is present in an amount of 1-20 parts for each 1-10 parts of terpolymer.

The method of producing the coated tablet according to the invention comprises the steps of dissolving the terpolymer in a solvent, preparing a suspension or solution of the pore-creating substance, providing a pharmaceutical tablet, combining the suspension or solution of pore-creating substance and solvent solution of the terpolymer to form a coating fluid, applying the coating fluid in the form of a solution or suspension to the tablet, and drying the coating fluid on the tablet to provide a terpolymer-coated tablet having the water-soluble pore-creating substance randomly distributed within the coating.

Detailed description of the invention

Preferably the terpolymer contains 80-95% weight per weight of polyvinyl chloride, 1 to 19% weight per weight of polyvinyl acetate and 1 to 10% weight per weight of polyvinyl alcohol. In this context it can be mentioned that a coating comprising a copolymer consisting of only polyvinyl chloride and polyvinyl acetate can be used but the adhesion properties to different kinds of drug containing tablet cores are not sufficiently good to avoid roughness of the tablet surface ("orange peel") and/or other problems related to poor adhesion. Furthermore, such a copolymer has not sufficient mechanical strength.

The pore-creating substance used according to the present invention should be highly water-soluble, pharmacologically acceptable and essentially free from own pharmacological effects in the amounts used. Especially preferred as pore-creating substance is saccarose (sucrose). Other substances which may be used include polyvinyl pyrrolidone, polyethylene glycol 1500, 4000 or 6000 and sodium chloride.

The ratio pore-creating agent to terpolymer depends on the desired dissolution rate and time and can be decided in each separate case from simple experiments in the laboratory. Generally it can be said that in order to get practically useful dissolution from tablets for oral use the ratio should vary between 1 to 5, preferably between 1.5 and 3.

The pore-creating substance is preferably but not necessary insoluble in the solvent used for coating the tablets.

The particle size of the pore-creating substance may vary between 0.5 and 50 millimicrons.

Preferably a plasticizer is also present in the terpolymer. The amount of this plasticizer may vary between 0.1 and 4% weight by weight of the coating fluid. Examples of suitable plasticizers are acetyltributylcitrate, polyethylene glycol, blown castor oil and glyceryl triacetate. Furthermore, the coating may include sodium bicarbonate as stabilizing agent.

Depending on the size and area of the tablet the coating weight may vary between 10 and 170 mg per tablet and the coating thickness may vary between 25 and 300 μm, preferably 50 and 200 μm.

According to the present invention the drug included in the core could be almost any drug that can be orally administered. In order to improve the solubility properties of the drugs the core may include conventionally used additives such as buffering agent, e.g. sodium bicarbonate or citric acid.

Examples of drugs that suitably are administered in the form of sustained and constant release preparations according to the present invention include, but are not limited to, e.g. phenylpropanolamine, cefalosporin (Cefaclorum), bensodiazepine derivatives, potassium chloride, sodium salicylate, choline theophyllinate, acetaminophen, acetylcystein, terbutaline, dextromethorphan, ascorbic acid, diltiazem, noscapine, oxybutynin, ibuprophen, urapidil, melperone, salbutamol, cimetidine, chlorpheniramine maleate, propanolol, L-dopa, piracetam, isosorbide dinitrate, indomethacin, zinc sulfate, diclofenac, litium sulfate, ferrous sulfate, pseudoephedrine, sodium cromoglycate, sodium PAS and meclomen.

The starting preparations are produced in the following manner:

1) A terpolymer containing (w/w%) 80-95% PVC (polyvinylchloride), 1-19% PVAC (polyvinylacetate), and 1-10% PVOH (polyvinylalcohol) is dissolved in a solvent, e.g. acetone, methylenechloride, methylethylketone, or mixtures of acetone and ethanol, acetone and methylenechloride, or the like.

2) A suspension or solution of the pore-creating substance is produced as follows:
The pore-creating particles are ground either by dry milling in a ball mill or by wet-milling in a glass bead milling device to a defined particle size, preferably between 0.5 μm and 50 μm. The particles are dispersed in solvents or mixtures or solvents, such as those previously mentioned, and mixed with the terpolymer solution.

The ratio between pore-creating substance and terpolymer in the coating fluid is as previously described for the ratio in the coating. The coating fluid may, as previously stated, include a plasticizer and sodium bicarbonate.

The coating fluid, in the form of a solution or suspension, is then applied on drug-containing cores by conventional coating procedure. Examples of such coating procedures are pan coating, manual or spray-coating, Würster coating, and other fluid-bed coating procedures. Coloring matter can also be incorporated in the coating fluid, and insoluble coloring materials are preferred.

A second coating can be applied, and may contain one or more same or different drugs, for which a rapid release is desirable. This coating fluid is preferably a water-based sugar coating and, therefore, a seal coating between the latter and the terpolymer membrane coating is frequently necessary or desirable.

The invention is further illustrated by the following examples.

Example 1

Coating on tablets containing potassium chloride 1 gram.

Composition of the coating fluid:

| | grams |
|---|---|
| Terpolymer (PVC)M (PVAC)N (PVOH)O, wherein PVC is polyvinylchloride, PVAC is polyvinylacetate, and PVOH is polyvinylalcohol, and wherein M = 31, N = 1 and O = 2 | 160 |
| Micronized powdered saccharose (particle size 1–10 μm) | 409 |
| Acetyl tributyl citrate | 35.6 |
| Blown castor oil | 26.9 |
| Sodium bicarbonate | 15 |
| Acetone ad | 4400 |

The coating process is performed in a coating pan and the coating fluid is sprayed onto the tablets with an airless spray-coating device. Five thousand tablets are coated and the average membrane weight is 60 mg per tablet.

The following table discloses that the dissolution rate of the drug is essentially constant during 8 hours and essentially independent of pH.

Table I

| Test Fluid | Percent drug released time (hours) | | | | | | | |
|---|---|---|---|---|---|---|---|---|
| | 1 | 2 | 3 | 4 | 5 | 6 | 7 | 8 |
| Intestinal juice | 18 | 32 | 50 | 64 | 77 | 89 | 97 | 100 |
| Deionized water | 12 | 30 | 45 | 62 | 73 | 86 | 95 | 95 |
| Gastric juice | 10 | 24 | 40 | 51 | 67 | 80 | 90 | 95 |

Example 2

Coating applied on tablets containing 500 mg of sodium salicylate

Composition of the coating fluid:

| | grams |
|---|---|
| Terpolymer (PVC)M, (PVAC)N, (PVOH)O, M = 31, N = 1 and 0 = 2 | 160 |
| Micronized saccharose (particle size 1–10 μm) | 409 |
| Acetyl tributyl citrate | 17.8 |
| Sodium bicarbonate | 15 |
| Acetone ad | 4400 |

The coating weight is 50 mg per tablet and the coating procedure is the same as in Example 1.

As diclosed in the following Table II this formulation is an example of how the inherent pH dependency of the drug active substance can be used in order to obtain a desired dissolution rate at a certain pH.

**Table II**

| pH of test fluid | Percent drug released Time (hours) | | | | | | |
|---|---|---|---|---|---|---|---|
| | 1 | 2 | 3 | 4 | 5 | 6 | 24 |
| 1 | 4 | 8 | 16 | 22 | 30 | 36 | 98 |
| 3 | 28 | 48 | 65 | 77 | 88 | 94 | 99 |
| 5 | 29 | 51 | 69 | 79 | 87 | 92 | 99 |
| 7,4 | 30 | 50 | 68 | 80 | 90 | 93 | 99 |

The dissolution rate of the drug will be about four times slower in the stomach than in the intestine and the solid drug substance does not get into direct contact with the mucous membranes. Thus the local irritating effect of sodium salicylate on the gastric mucosa is minimized.

Example 3

Noscapine HCl is a weak base and has low solubility in natural and basic media, while the solubility in acidic media is high. Below, two tablet compositions are listed, one buffered B, and one without buffer A. Both tablet batches are coated with the same coating and the dissolution rate is determined according to USP XX.

**Tablet composition**

| | A | B |
|---|---|---|
| Noscapine HCL | 50 mg | 50 mg |
| Acidic Sodiumcitrate | – | 50 mg |
| Citric Acid | – | 50 mg |
| Powdered Sucrose | 242.5 mg | 142.5 mg |
| PVP 25 | 15 mg | 15 mg |

**Coating composition**

| | |
|---|---|
| Film-forming polymer | 6.96 mg |
| Micronized sucrose | 22.26 mg |
| Acetyltributylcitrate | 0.30 mg |
| Blown Castor Oil | 0.24 mg |

Example 4

Meclomen, Mefenanic acid, is a weak acid with a pKa of 10 which means that the drug is practically insoluble in physiological fluids having pH between 1 and 8. The tablet was buffered with sodium bicarbonate and the internal pH rises to about 10.5 where Mefenamic acid is freely soluble. As in the earlier experiments two formulations were manufactured, one containing buffer and one without.

**Tablet composition**

|  | A | B |
|---|---|---|
| Mefenamic acid Sodium salt | 164 mg | 164 mg |
| Sodium carbonate | – | 13 mg |
| Strearic acid/Talc 50% | 20 mg | 20 mg |
| Polyethylene glycol 6000 | 50 mg | 50 mg |
| Powered Sucrose | 126 mg | 113 mg |

**Coating composition**

| Micronized sucrose | 17.9 mg |
|---|---|
| Film-forming polymer | 6.0 mg |
| Acetyltributylcitrate | 0.25 mg |
| Blown Castor oil | 0.2 mg |

Example 5

Melperone, a weak base, has a $pK_6$ of 8.8 and shows pH-dependent release rate. Two formulations, one containing citric acid as buffering agent, and one without, were manufactured.

**Tablet formulation**

|  | A | B |
|---|---|---|
| Melperone HCl | 50 mg | 50 mg |
| Acetylglycinamide | 95 mg | 95 mg |
| PVP 25 Kollidon | 5 mg | 5 mg |
| Glycerol | 1 mg | 1 mg |
| Citric acid | – | 25 mg |
| Lactose | 97.5 | 97.5 mg |
| Stearic acid/Talc, 50% | 10 mg | 10 mg |
| Avicel PH | 62.5 mg | 37.5 mg |

**Coating formulation**

| Micronized sucrose | 21.3 mg |
|---|---|
| Film-forming polymer | 6.85 mg |
| Acetyltributylcitrate | 0.32 mg |
| Blown Castor oil | 0.24 mg |

The following Table III discloses the differences in dissolution rate for the preparations with and without buffer according to the examples 3-5.

**Table III**

Dissolved amount after four hours:

|  | Meclomen | | Melperone | | Noscapine | |
|---|---|---|---|---|---|---|
|  | pH 10 | pH 7.4 | pH 7.4 | pH 8.0 | pH 1 | pH 7.4 |
| Buffered | 91% | 84% | 74% | 78% | 73% | 69% |
| Whithout Buffer | 91% | 24% | 34% | 78% | 75% | 1.2% |

6

Example 6

Coated Choline Theophyllinate Tablets

Coating applied on tablets containing 270 mg of choline theo phyllinate.
Composition of the coating fluid:

|  | grams |
|---|---|
| Terpolymer (PVC)M, (PVAC)N, (PVOH)O, M = 31, N = 1 and O = 2 | 130 |
| Micronized saccharose (particle size 1–10 μm) | 409 |
| Acetyltributyl citrate | 14.3 |
| Blown castor oil | 10.9 |
| Sodium bicarbonate | 15 |
| Acetone ad | 4400 |

The weight of the membrane is 40 mg per tablet and the coating procedure is the same as in Example 1.

Example 7

Coated bensodiazepine tablets

Coating applied on tablets containing 6 mg nitrazepame (bensodiazepinderivative)

| Tablet: |  |
|---|---|
| Nitrazepame | 4 mg |
| Powdered sucrose | 120 mg |
| Polyethylen oxide 6000 | 110 mg |
| Polyvinylpyrrolidone | 5 mg |
| Magnesium stearate | 2 mg |

The ingredients except for Mg-stearate were mixed and moistened with ethanol. After drying Mg-stearate was added and the powder was compressed to tablets.

| Coating: |  |
|---|---|
| Terpolymer according to Example 1 | 9.8 mg |
| Acetyltributyl citrate | 1.87 mg |
| Blown castor oil |  |
| Micronized sucrose | 23 mg |
| Acetone | 530 mg |

Example 8

Coated Paracetamole Tablets

Coating applied on tablets containing 500 mg of paracetaminophenol

|  | grams |
|---|---|
| Terpolymer (PVC)M, (PVAC)N, (PVOH)O, M = 31, N = 1 and O = 2 | 120 |
| Polyethyleneglycol 6000 (pore-creating substance) | 410 |
| Acetyl tributyl citrate | 12 |
| Acetone ad | 4400 |

Example 9

Terpolymer Variation

Coating applied on tablets containing 1 g of potassium chloride.
Composition of the coating fluid:

|  | grams |
|---|---|
| Terpolymer (PVC)M, (PVAC)N, (PVOH)O, M = 100, N = 1 and O = 8 | 160 |
| Micronized powdered saccharose (particle size 1–10 μm) | 409 |
| Acetyltributyl citrate | 35.6 |
| Blown castor oil | 26.4 |
| Sodium bicarbonate | 15 |
| Acetone ad | 4400 |

The weight of the dried membrane was 60 mg per tablet and the coating procedure was the same as in Example 1.

**Claims**

1. A sustained-release coated pharmaceutical tablet exhibiting a substantially constant dissolution rate during a major portion of its dissolution time when exposed to gastrointestinal fluids, and comprising a drug-containing tablet and a coating surrounding the same, this coating essentially consisting of a polymer, which is insoluble in water and gastro-intestinal fluids and a watersoluble pore-creating substance randomly distributed in the polymer, characterized in that the polymer is a terpolymer of vinylchloride, vinylacetate and vinylalcohol, that the ratio pore-creating substance to terpolymer varies between 0,1 and 20, that the pore-creating substance consists of particles, which are essentially insoluble in the solvent used for coating the drug-containing tablet and that the pore-creating agent is substantially pharmacologically inactive in the amount used.

2. A coated tablet according to claim 1, wherein the terpolymer contains 80 to 95% weight by weight of vinylchloride, 1 to 19% weight by weight of vinylacetate, and 1 to 10% weight by weight of vinylalcohol.

3. A coated tablet according to claim 2, wherein the pore-creating substance is a water-soluble substance selected from the group of saccharose, sodium chloride, polyvinylpyrrolidone and a polyethylene glycol.

4. A coated tablet according to any of the claims 1–3, wherein the ratio pore-creating substance to terpolymer varies between 1 and 5 and preferably between 1.5 and 3.

5. A coated tablet according to any of the preceding claims, wherein the tablet also includes a buffering agent.

6. A coated tablet according to any of the preceding claims, wherein the coating also includes a plasticizer selected from acetyltributylcitrate, polyethylene glycol, blown castor oil and glyceryl triacetate.

7. A coated tablet according to claim 6, wherein the plasticizer is present in a concentration of 0.1 to 4% weight by weight of coating fluid.

8. Method of producing a coated tablet exhibiting a substantially constant dissolution rate during a major portion of its dissolution time when exposed to gastrointestinal fluids, and comprising a drug-containing tablet and a coating surrounding the same, wherein the coating essentially consists of a polymer, which is insoluble in water and gastrointestinal fluids and a watersoluble, pore-creating substance randomly distributed in the polymer, characterized in that the polymer is a terpolymer of vinylchloride, vinylacetate and vinylalcohol, that the ratio pore-creating substance to terpolymer varies between 0,1 and 20, that the pore-creating substance consists of particles, which are essentially insoluble in the solvent used for coating and that the pore-creating substance is essentially inactive in the amount used, comprising the steps of dissolving the said terpolymer in a solvent, preparing a suspension of the pore-creating substance, providing a pharmaceutical tablet, combining the suspension of pore-creating substance and solvent solution of the terpolymer to form a coating fluid, applying the coating fluid in the form of suspension to the tablet, and drying the coating fluid on the tablet to provide a terpolymer-coated tablet having watersoluble pore-creating substance randomly distributed within the coating or membrane.

9. A method according to claim 8, wherein the terpolymer contains 80 to 95% weight by weight of vinylchloride, 1 to 19% weight by weight of vinylacetate, and 1 to 10% weight by weight of polyvinylalcohol.

10. A method according to claim 8 or 9, wherein the pore-creating substance is a water-soluble substance selected from the group consisting of saccharose, sodium chloride, polyvinylpyrrolidone and a polyethylene glycol.

11. A method according to any of the claims 8–10, wherein the ratio pore-creating substance to terpolymer varies between 1 and 5 and preferably between 1.5 and 3.

12. A method according to any of the claims 8–11, wherein the tablet also includes a buffering agent.

13. A method according to any of the claims 8–12, wherein a plasticizer is also present in the terpolymer.

14. A method according to claim 13, wherein the plasticizer is present in a concentration of 0.1 to 4% weight by weight of coating fluid, preferably selected from acetyltributylcitrate, polyethylene glycol, blown castor oil, and glyceryl triacetate.

## Patentansprüche

1. Beschichtete pharmazeutische Tablette mit verlängerter Freisetzungsdauer und einer im wesentlichen konstanten Lösungsgeschwindigkeit während eines Großteils ihrer Lösungszeit, wenn sie den gastrointestinalen Flüssigkeiten ausgesetzt ist, bestehend aus einer den Arzneistoff enthaltenden Tablette und einem dieselbe umschließenden Überzug, welcher Überzug im wesentlichen aus einem in Wasser und in den gastrointestinalen Flüssigkeiten unlöslichen Polymer und einer willkürlich im Polymer verteilten, wasserlöslichen, porenbildenden Substanz besteht, dadurch gekennzeichnet, daß das Polymer ein Terpolymer aus Vinylchlorid, Vinylacetat und Vinylalkohol ist, daß das Verhältnis von porenbildender Substanz zu Terpolymer zwischen 0,1 und 20 variiert, daß die porenbildende Substanz aus Teilchen besteht, die in dem für die Beschichtung der den Arzneistoff enthaltenden Tablette verwendeten Lösungsmittel im wesentlichen unlöslich sind, und daß das porenbildende Mittel in den angewendeten Mengen pharmakologisch im wesentlichen inaktiv ist.

2. Beschichtete Tablette nach Anspruch 1, dadurch gekennzeichnet, daß das Terpolymer 80 bis 95 Gew.-% Vinylchlorid, 1 bis 19 Gew.-% Vinylacetat und 1 bis 10 Gew.-% Vinylalkohol enthält.

3. Beschichtete Tablette nach Anspruch 2, dadurch gekennzeichnet, daß die porenbildende Substanz eine wasserlösliche Substanz, ausgewählt aus der Gruppe Saccharose, Natriumchlorid, Polyvinylpyrrolidon und einem Polyethylenglykol, ist.

4. Beschichtete Tablette nach einem der Ansprüche 1 bis 3, dadurch gekennzeichnet, daß das Verhältnis von porenbildender Substanz zu Terpolymer zwischen 1 und 5, vorzugsweise zwischen 1,5 und 3, variiert.

5. Beschichtete Tablette nach einem der vorhergehenden Ansprüche, dadurch gekennzeichnet, daß die Tablette auch ein Puffermittel enthält.

6. Beschichtete Tablette nach einem der vorhergehenden Ansprüche, dadurch gekennzeichnet, daß der Überzug auch einen Weichmacher, ausgewählt aus Acetyltributylcitrat, Polyethylenglykol, geblasenes Rizinusöl und Glycerintriacetat, enthält.

7. Beschichtete Tablette nach Anspruch 6, dadurch gekennzeichnet, daß der Weichmacher in einer Konzentration von 0,1 bis 4 Gew.-% des Gewichtes der Beschichtungsflüssigkeit zugegen ist.

8. Verfahren zur Herstellung einer beschichteten pharmazeutischen Tablette mit im wesentlichen konstanter Lösungsgeschwindigkeit während eines Großteils ihrer Lösungszeit, wenn sie den gastrointestinalen Flüssigkeiten ausgesetzt ist, bestehend aus einer den Arzneistoff enthaltenden Tablette und einem dieselbe umschließenden Überzug, welcher Überzug im wesentlichen aus einem in Wasser und in den gastrointestinalen Flüssigkeiten unlöslichen Polymer und einer willkürlich im Polymer verteilten, wasserlöslichen, porenbildenden Substanz besteht, dadurch gekennzeichnet, daß das Polymer ein Terpolymer aus Vinylchlorid, Vinylacetat und Vinylalkohol ist, daß das Verhältnis von porenbildender Substanz zu Terpolymer zwischen 0.1 und 20 variiert, daß die porenbildende Substanz aus Teilchen besteht, die in dem für die Beschichtung der den Arzneistoff enthaltenden Tablette verwendeten Lösungsmittel im wesentlichen unlöslich sind, und daß das porenbildende Mittel in den angewendeten Mengen im wesentlichen inaktiv ist, umfassend die Stufen der Lösung des Terpolymers in einem Lösungsmittel, Herstellung einer Suspension der porenbildenden Substanz. Erzeugung einer pharmazeutischen Tablette. Vereinigung der Suspension der porenbildenden Substanz und der Lösungsmittellösung des Terpolymers, um eine Beschichtungsflüssigkeit zu bilden. Aufbringung der Beschichtungsflüssigkeit in Suspensionsform auf die Tablette und Trocknung der Beschichtungsflüssigkeit auf der Tablette, um eine Terpolymer-beschichtete Tablette mit einer im Überzug oder in der Membran willkürlich verteilten porenbildenden Substanz zu bilden.

9. Verfahren nach Anspruch 8, dadurch gekennzeichnet, daß das Terpolymer 80 bis 95 Gew.-% Vinylchlorid, 1 bis 19 Gew.-% Vinylacetat und 1 bis 10 Gew.-% Vinylalkohol enthält.

10. Verfahren nach Anspruch 8 oder 9, dadurch gekennzeichnet, daß die porenbildende Substanz eine wasserlösliche Substanz, ausgewählt aus der Gruppe Saccharose, Natriumchlorid, Polyvinylpyrrolidon und einem Polyethylenglykol, ist.

11. Verfahren nach einem der Ansprüche 8 bis 10, dadurch gekennzeichnet, daß das Verhältnis von porenbildender Substanz zu Terpolymer zwischen 1 und 5, vorzugsweise zwischen 1,5 und 3, variiert.

12. Verfahren nach einem der Ansprüche 8 bis 11, dadurch gekennzeichnet, daß die Tablette auch ein Puffermittel enthält.

13. Verfahren nach einem der Ansprüche 8 bis 12, dadurch gekennzeichnet, daß im Terpolymer auch ein Weichmacher zugegen ist.

14. Verfahren nach Anspruch 13, dadurch gekennzeichnet, daß der Weichmacher in einer Konzentration von 0,1 bis 4 Gew.-% des Gewichtes der Beschichtungsflüssigkeit zugegen und vorzugsweise aus Acetyltributylcitrat, Polyethylenglykol, geblasenem Rizinusöl und Glycerintriacetat ausgewählt ist.

## Revendications

1. Comprimé pharmaceutique enrobé, à libération prolongée, présentant une vitesse de dissolution pratiquement constante pendant la plus grande partie de son temps de dissolution lorsqu'il est mis en contact avec les fluides gastro-intestinaux, et comprenant un comprimé contenant un médicament et un enrobage entourant ce comprimé, cet enrobage consistant essentiellement en un polymère, qui est insoluble dans l'eau et les fluides gastro-intestinaux, et en une substance hydrosoluble porogène distribuée d'une manière aléatoire dans le polymère, caractérisé en ce que le polymère est un terpolymère de chlorure de vinyle, d'acétate de vinyle et d'alcool vinylique, le rapport de la substance porogène au terpolymère varie de 0,1 à 20, la substance porogène consiste en particules qui sont pratiquement insolubles dans le solvant utilisé pour l'enrobage du comprimé contenant un médicament et l'agent porogène est pratiquement pharmacologiquement inactif en la quantité utilisée.

2. Comprimé enrobé suivant la revendication 1, dans lequel le terpolymère contient 80 à 95% en poids/poids de chlorure de vinyle, 1 à 19% en poids/poids d'acétate de vinyle et 1 à 10% en poids/poids d'alcool vinylique.

3. Comprimé enrobé suivant la revendication 2, dans lequel la substance porogène est une substance hydrosoluble choisie dans le groupe comprenant le saccharose, le chlorure de sodium, la polyvinylpyrrolidone et un polyéthylèneglycol.

4. Comprimé enrobé suivant l'une quelconque des revendications 1 à 3, dans lequel le rapport de la substance porogène au terpolymère varie de 1 à 5 et, de préférence, de 1,5 à 3.

5. Comprimé enrobé suivant l'une quelconque des revendications précédentes, dans lequel le comprimé renferme également un tampon.

6. Comprimé enrobé suivant l'une quelconque des revendications précédentes, dans lequel l'enrobage renferme également un plastifiant choisi entre le citrate d'acétyltributyle, un polyéthylèneglycol, l'huile de ricin soufflée et le triacétate de glycéryle.

7. Comprimé enrobé suivant la revendication 6, dans lequel le plastifiant est présent en une concentration de 0,1 à 4% en poids/poids du fluide d'enrobage.

8. Procédé de production d'un comprimé enrobé présentant une vitesse de dissolution pratiquement constante pendant la plus grande partie de son temps de dissolution lors de sa mise en contact avec les fluides gastro-intestinaux et comprenant un comprimé contenant un médicament et un enrobage entourant le comprimé, dans lequel le revêtement consiste essentiellement en un polymère qui est insoluble dans l'eau et les fluides gastro-intestinaux, et en une substance porogène hydrosoluble distribuée de manière aléatoire dans le polymère, caractérisé en ce que le polymère est un terpolymère de chlorure de vinyle, d'acétate de vinyle et d'alcool vinylique, le rapport de la substance porogène au terpolymère varie de 0,1 à 20, la substance porogène consiste en particules qui sont pratiquement insolubles dans le solvant utilisé pour l'enrobage et la substance porogène est pratiquement inactive en la quantité utilisée, comprenant les étapes consistant à dissoudre ledit terpolymère dans un solvant, à préparer une suspension de la substance porogène, à produire un comprimé pharmaceutique, à mélanger la suspension de substance porogène et la solution, dans un solvant, du terpolymère pour former un fluide d'enrobage, à appliquer le fluide d'enrobage sous forme d'une suspension au comprimé et à sécher le fluide d'enrobage sur le comprimé pour produire un comprimé enrobé avec le terpolymère, ayant une substance porogène hydrosoluble distribuée de manière aléatoire à l'intérieur de l'enrobage ou de la membrane.

9. Procédé suivant la revendication 8, dans lequel le terpolymère contient 80 à 95% en poids/poids de chlorure de vinyle, 1 à 19% en poids/poids d'acétate de vinyle et 1 à 10% en poids/poids d'alcool polyvinylique.

10. Procédé suivant la revendication 8 ou 9, dans lequel la substance porogène est une substance hydrosoluble choisie dans le groupe comprenant le saccharose, le chlorure de sodium, la polyvinylpyrrolidone et un polyéthylèneglycol.

11. Procédé suivant l'une quelconque des revendications 8 à 10, dans lequel le rapport de la substance porogène au terpolymère varie de 1 à 5 et, de préférence, de 1,5 à 3.

12. Procédé suivant l'une quelconque des revendications 8 à 11, dans lequel le comprimé renferme également un tampon.

13. Procédé suivant l'une quelconque des revendications 8 à 12, dans lequel un plastifiant est également présent dans le terpolymère.

14. Procédé suivant la revendication 13, dans lequel le plastifiant est présent en une concentration de 0,1 à 4% en poids/poids du fluide de revêtement, et est choisi de préférence entre le citrate d'acétyltributyle, le polyéthylèneglycol, l'huile de ricin soufflée et le triacétate de glycéryle.